(19) Europäisches Patentamt

European Patent Office

Office européen des brevets

(11) **EP 1 369 101 A1**

(12) **EUROPEAN PATENT APPLICATION**
published in accordance with Art. 158(3) EPC

(43) Date of publication:
**10.12.2003 Bulletin 2003/50**

(51) Int Cl.⁷: **A61K 7/00**, A61K 7/40,
A61K 7/42

(21) Application number: **02702880.2**

(22) Date of filing: **11.03.2002**

(86) International application number:
**PCT/JP02/02259**

(87) International publication number:
**WO 02/074260 (26.09.2002 Gazette 2002/39)**

(84) Designated Contracting States:
**AT BE CH CY DE DK ES FI FR GB GR IE IT LI LU
MC NL PT SE TR**

(30) Priority: **16.03.2001 JP 2001076884**

(71) Applicant: **SHISEIDO COMPANY LIMITED
Chuo-ku, Tokyo 104-8010 (JP)**

(72) Inventors:
• **NAKAMURA, Tadashi,
C/O SHISEIDO RESEARCH CENTER
Yokohama-shi, Kanagawa 224-8558 (JP)**
• **TAKASU, Emiko,
C/O SHISEIDO RESEARCH CENTER
Yokohama-shi, Kanagawa 224-8558 (JP)**

(74) Representative: **Santarelli
14, avenue de la Grande Armée
75017 Paris (FR)**

(54) **COSMETIC W/O/W EMULSION PREPARATION**

(57) A W/O/W type emulsified cosmetic composition contains a W/O type emulsion dispersed in an external aqueous phase, wherein the W/O type emulsion contains an emulsifier having an HLB of not more than 7 and an electrolyte and wherein the external aqueous phase contains an alkyl-modified carboxyvinyl polymer.

**Description**

TECHNICAL FIELD

The present invention relates to a W/O/W type emulsified cosmetic composition. More particularly, it relates to a W/O/W type emulsified cosmetic composition superior in feelings in use such as feeling upon application to the skin, affinity, moistness and having a good stability with the elapse of time.

BACKGROUND ART

In the past, O/W type and W/O type cosmetic compositions have been generally used as emulsified cosmetic compositions, but multi-type emulsions, that is, W/O/W type emulsified cosmetic compositions, can impart a feeling in use unknown in W type and W/O type emulsified cosmetic compositions. For example, those designed to give a refreshingness in use like a phase inversion when applied to the skin etc. are known.

However, cosmetic compositions having a refreshingness in use like a phase inversion when applied to the skin suffer from the problems that a W/O/W type preparation is unstable as a system and is inferior in the stability with elapse of time. Therefore, a W/O/W type preparation excellent in stability with elapse of time and feeling in use has been sought.

As a method for obtaining a stable W/O/W type complex emulsion, for example, Japanese Unexamined Patent Publication (Kokai) No. 2-95433 discloses a method for preparing an internal phase W/O type microemulsion and a W/O/W complex emulsion using an SPG film. Japanese Unexamined Patent Publication (Kokai) No. 59-127646 and Japanese Unexamined Patent Publication (Kokai) No. 60-193529 disclose methods for preparing W/O type emulsions using specific bentonite or metal soaps, then preparing O/W type complex emulsions using N-long chain acyl acidic amino acid monosalt or a hydrophilic nonionic surfactant. Further, Japanese Unexamined Patent Publication (Kokai) No. 11-33391 discloses a technology for improving the stability of a W/O/W type complex emulsion by dispersing a W/O type emulsion in an external aqueous phase using an alkyl-modified carboxyvinyl polymer.

However, none of the W/O/W preparations up to now have reached a level sufficiently capable of satisfying the requirements for use and stability with elapse of time.

DISCLOSURE OF THE INVENTION

Accordingly, the objects of the present invention are to solve the above problems in the prior art and to provide a W/O/W type emulsified cosmetic composition excellent in stability with elapse of time, while having refreshingness in use like a phase inversion when applied to the skin, smoothness and moistness.

The present inventors engaged in repeated in-depth research and, as a result, found that by formulating an electrolyte into an internal aqueous phase, using a specific emulsifier for emulsification, and dispersing the W/O type emulsion thus prepared into an external aqueous phase including an alkyl-modified carboxyvinyl polymer to prepare a W/O/W type complex emulsion, it is possible to solve the above problems, whereby the present invention was completed.

That is, the present invention provides a W/O/W type emulsified cosmetic composition comprising a W/O type emulsion dispersed in an external aqueous phase, wherein the W/O type emulsion contains an 7 emulsifier having an HLB of not more than 7 and an electrolyte and wherein the external aqueous phase contains an alkyl-modified carboxyvinyl polymer.

Further, the present invention provides a W/O/W type emulsified cosmetic composition wherein the 7 emulsifier having an HLB of not more than 7 is at least one member selected from the group consisting of polyhydroxystearic acid esters of polyhydric alcohols and polyether-modified silicone-based surfactants.

Further, the present invention provides a W/O/W type emulsified cosmetic composition wherein the electrolyte is at least one member selected from the group consisting of amino acids, L-ascorbic acids and derivatives thereof.

MODE OF WORKING THE INVENTION

The present invention will now be explained in detail.

The W/O/W type emulsified cosmetic composition is structured as a W/O type emulsion dispersed in an external aqueous phase.

In the present invention, the W/O type emulsion forming the internal phase contains an electrolyte in the innermost aqueous phase. The electrolyte is not particularly limited, but amino acids, L-ascorbic acids, or derivatives thereof are preferably used. As these derivatives, salts, esters, etc. may be mentioned.

The amino acid or the derivative thereof is suitably selected from amino acids and their salts known as general food additives and pharmacological drugs. For example, L-alanine, β-alanine, L-arginine hydrochloride, L-aspartic acid 1-hydrate, L-aspartic acid, L-chitolulin, L-glutamic acid, L-glutamic acid hydrochloride, L-glutamine, glycine, trimethyl glycine, L-histidine, L-histidine hydrochloride 1-hydrate, L-hydroxyproline, L-isoleucine, L-leucine, L-lysine, L-lysine hydrochloride, L-ornithine hydrochloride, L-proline, L-phenylalanine, L-serine, L-threonine, L-tryptophan, L-tyrosine, L-valine, L-dopa, L-α-aminobutyric acid, etc. may be mentioned. Among these in particular, glycine, L-serine, L-alanine, L-proline, trimethylglycine, etc. are preferably used.

Further, as a metal salt of the above amino acids, a monovalent metal salt such as a sodium, potassium, or lithium salt, a bivalent metal salt such as a calcium or magnesium salt etc. may be used. Among these, sodium L-aspartate 1-hydrate, potassium aspartate 2-hydrate, sodium L-glutamate 1-hydrate, potassium L-glutamate 1-hydrate, etc. may be particularly mentioned as preferable.

L-ascorbic acid is generally called "vitamin C". As its derivatives, L-ascorbyl monoalkyl esters such as L-ascorbyl monostearate, L-ascorbyl monopalmitate, L-ascorbyl monooleate; L-ascorbyl monoesters such as L-ascorbyl monophosphate ester, L-ascorbyl-2-sulfuric acid esters; L-ascorbyl dialkyl esters such as L-ascorbyl distearate, L-ascorbyl dipalmitate, L-ascorbyl dioleate; L-ascorbyl trialkyl esters such as L-ascorbyl tristearate, L-ascorbyl tripalmitate, L-ascorbyl trioleate; L-ascorbyl triesters such as L-ascorbyl triphosphoric acid esters; L-ascorbyl glucosides such as L-ascorbyl-2-glucoside; etc. may be mentioned. Among these, L-ascorbic acid, L-ascorbyl phosphoric acid esters, L-ascorbyl-2-sulfuric acid esters, L-ascorbyl-2-glucoside, etc. are particularly preferably used.

The electrolyte used is preferably one or more of the above-mentioned amino acids, L-ascorbic acid and their derivatives.

The amounts of the electrolyte formulated is preferably 0.01 to 5% by weight in the total weight of the cosmetic composition of the present invention, in particular 0.1 to 3% by weight. If the amount formulated is too small, no improvement in the feeling in use is obtained, while if the amount formulated is too large, the storage with the elapse of time becomes poorer in some cases.

The oil ingredient contained in the oil phase is not particularly limited. For example, a liquid oil or fat, solid oil or fat, wax, hydrocarbon oil, higher fatty acid, higher alcohol, synthetic ester oil, silicone oil, etc. may be used, but the invention is not limited thereto.

As the liquid oil or fat, for example, avocado oil, primrose oil, turtle oil, macademia nut oil, corn oil, mink oil, olive oil, rapeseed oil, egg oil, sesame oil, persic oil, wheat germ oil, sasanqua oil, castor oil, linseed oil, safflower oil, cottonseed oil, perilla oil, soybean oil, peanut oil, teaseed oil, kaya oil, rice bran oil, Chinese wood oil, Japanese wood oil, jojoba oil, germ oil, triglycerin, glyceryl trioctanate, glyceryl triisopalmitate, etc. may be mentioned.

As the solid oil or fat, for example, cacao butter, coconut oil, horse fat, hydrogenated coconut oil, palm oil, beef tallow, sheep fat, palm kernal oil, hog fat, beef bone fat, Japan wax nut oil, hydrogenated oil, beef hoof fat, Japan wax, hydrogenated castor oil, etc. may be mentioned.

As the wax, for example, beeswax, candelilla wax, cotton wax, carnauba wax, bayberry wax, insect wax, spermaceti, montan wax, rice bran wax, lanolin, kapok wax, lanolin acetate, liquid lanolin, sugarcane wax, isopropyl lanolin fatty acid, hexyl laurate, hydrogenated lanolin, jojoba wax, hard lanolin, shellac wax, POE lanolin alcohol ether, POE lanolin alcohol acetate, POE cholesterol ether, polyethylene glycol lanolin fatty acid, POE hydrated lanolin alcohol ether, etc. may be mentioned.

As the hydrocarbon oil, for example, liquid paraffin, ozokerite, squalane, pristane, paraffin, ceresine, squalene, vaseline, microcrystalline wax, etc. may be mentioned.

As the higher fatty acid, for example, lauric acid, myristic acid, palmitic acid, isostearic acid, stearic acid, behenic acid, 12-hydroxystearic acid, undecylic acid, toluic acid, etc. may be mentioned.

As the higher alcohol, for example, cetyl alcohol, stearyl alcohol, behenyl alcohol, myristyl alcohol, cetostearyl alcohol, etc. may be mentioned.

As the synthetic ester oil, for example, isopropyl myristate, cetyl octanate, octyldodecyl myristate, isopropyl palmitate, butyl stearate, hexyl laurate, myristyl myristate, decyl oleate, hexyldecyl dimethyloctanate, cetyl lactate, myristyl lactate, lanolin acetate, isocetyl stearate, isocetyl isostearate, cholesteryl 12-hydroxystearate, ethylene glycol di-2-ethylhexylate, dipentaerythritol fatty acid ester, N-alkylglycol monoisostearate, neopentylglycol dicaproate, diisostearyl malate, glyceryl di-2-heptyl undecanoate, trimethylolpropane tri-2-ethylhexylate, trimethylolpropane triisostearate, pentanerythritol tetra-2-ethylhexylate, glyceryl tri-2-ethylhexylate, trimethylolpropane triisostearate, cetyl-2-ethylhexanoate, 2-ethylhexyl palmitate, glyceryl trimyristate, glyceride tri-2-heptylundecanoate, castor oil fatty acid methyl ester, oil oleate, cetostearyl alcohol, acetoglyceride, 2-heptylundecyl palmitate, diisobutyl adipate, N-lauroyl-L-glutamate-2-octyldodecyl ester, di-2-heptylundecyl adipate, ethyl laurate, di-2-ethylhexyl sebatate, 2-hexyldecyl myristate, 2-hexyldecyl palmitate, 2-hexyldecyl adipate, diisopropyl sebatate, 2-ethylhexyl succinate, ethyl acetate, butyl acetate, amyl acetate, triethyl citrate, etc. may be mentioned.

As the silicone oil, for example, chain polysiloxanes such as dimethyl polysiloxane, methylphenyl polysiloxane, methylhydrogen polysiloxane; and, cyclic polysiloxanes such as decamethyl polysiloxane, dodecamethyl polysiloxane, tetramethyltetrahydrogen polysiloxane, etc. may be mentioned.

The oil ingredient may be used alone or in any combination thereof.

The above internal aqueous phase and oil phase are emulsified with an emulsifier having an HLB of not more than 7 to form a W/O type emulsion. With an emulsifier having an HLB of more than 7, the hydrophilicity is high and a stable W/O type emulsion cannot be obtained, and therefore, this is not preferred. Note that the HLB is calculated by the Kawakami formula shown in the following equation 1.

$$HLB = 7+11.7 \cdot \log (MW/MO)$$

where MW indicates the molecular weight of the hydrophilic group part, while MO indicates the molecular weight of the lyophilic group part.

As or 7 emulsifier having an HLB of 7 or less, for example, sorbitan fatty acid esters such as sorbitan monolaurate, sorbitan monooleate, sorbitan moniisostearate, sorbitan tristearate; glyceryl fatty acid esters such as glyceryl monostearate, glyceryl monostearate, glyceryl monooleate; poloxyethylene hydrogenated castor oils such as POE(5) hydrogenated castor oil, POE(7.5)hydrogenated castor oil, POE(10)hydrogenated castor oil; polyether-modified silicone-based surfactants such as dimeticone copolyol, cetyl dimeticone copolyol, dimeticone copolyol cross polymer; polyoxyalkylenated glycol fatty acid ester-based surfactants; polyglyceryl fatty acid ester-based surfactants; polyhydric alcohol polyhydroxystearate esters, polyhydroxystearyl polyglycerin, cross-linked organopolysiloxane elastomers containing polyoxyethylene chains and/or polyoxypropylene chains, etc. may be mentioned.

Among these, the polyhydric alcohol polyhydroxystearate ester polyhydroxystearyl alkylenated glycol (for example, polyethyleneglycol dipolyhydroxystearate "Arlacel P135", made by ICI), polyhydroxystearyl polyglycerin ("DEHYMULUS PGPH", made by Henkel), the polyether-modified silicone-based surfactant dimeticone copolyol ("Silicone SC 9450", made by Shinetsu Chemical), a cross-linked organopolysiloxane elastomer containing polyoxyethylene chains and/or polyoxypropylene chains ("KSG 21", made by Shinetsu Chemical), etc. may be particularly preferably used.

The amount formulated of the emulsifier having an HLB of 7 or less is preferably 0.01 to 10% by weight of the total weight of the cosmetic composition of the present invention, preferably 0.1 to 7% by weight. If the amount formulated is too small, no improvement in the feeling in use is obtained, while if the amount formulated is too large, formation of a W/O/W type emulsion is sometimes unstable.

The W/O/W type emulsified cosmetic composition of the present invention contains an external aqueous phase in which an alkyl-modified carboxylvinyl polymer is formulated.

The alkyl-modified carboxyvinyl polymer capable of being used in the present invention acts as an emulsifier and thickener. The alkyl-modified carboxyvinyl polymer preferably has a molecular weight of 500,000 to 3,000,000. The alkyl-modified carboxyvinyl polymer is for example commercially available as "Carbopol 1342", "Pemulen TR-1", "Pemulen TR-2" (above all made by B.F. Goodrich), etc., which may be preferably used.

The amount formulated of the alkyl-modified carboxyvinyl polymer is preferably 0.01 to 2% by weight of the total weight of the cosmetic composition of the present invention. If the amount formulated is too small, the stability deteriorates, while if the amount formulated is too large, a grating feeling and twisting sometimes occur.

The W/O/W type emulsified cosmetic composition of the present invention may also contain, if desired, in addition to the above essential ingredients, any added ingredients normaly formulated into cosmetic compositions to an extent not detracting from the effects of the present invention. As such additional ingredients, for example, humectants such as polyethylene glycol, glyceryl-1,3-butyleneglycol, erythritol, sorbitol, xylitol, maltitol; thickeners such as cellulose, hydroxyethyl cellulose, hydroxypropyl cellulose, methyl hydroxypropylcellulose, methyl cellulose, carboxymethyl cellulose, quince seed, carrageenan, pectin, mannan, curdlan, chondroitin sulfate, starch, galactan, dermatan sulfate, glycogen, gum arabic, heparin sulfate, hyaluronic acid, sodium hyaluronate, traganth gum, keratan sulfate, chondroitin, xanthan gum, mucoitin sulfate, hydroxyethyl guar gum, carboxymethyl guar gum, guar gum, dextran, kerato sulfate, locust bean gum, succinoglucan, charonic acid, chitin, chitosan, carboxymethyl chitin, agar; alcohols such as ethanol; antioxidants such as butylhydroxytoluene, tocopherol, phytin; benzoic acid-based UV absorbants such as benzoic acid, salicylic acid, sorbic acid, paraoxybenzoic acid alkyl ester, hexachlorophene, and other antibactericides; para-aminobenzoic acid (hereinafter abbreviated as "PABA"), PABA monoglyceryl ester, N,N-dipropoxy-PABA-ethyl ester, N,N-diethoxy-PABA-ethyl ester, N,N-dimethyl-PABA-methyl ester, N,N-dimethyl-PABA-ethyl ester, N,N-dimethyl-PABA-butyl ester, N,N-dimethyl-PABA 2-ethylhexyl ester; anthranilic acid-based UV absorbants such as homomentyl-N-acetylanthranilate; salicylic acid-based UV absorbants such as amyl salicylate, mentyl salicylate, homomentyl salicylate, octyl salicylate, phenyl salicylate, benzyl salicylate, p-isopropanol phenyl salicylate; cinnamic acid-based UV absorbants such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate(2-ethylhexyl-p-methoxy cinnamate), 2-ethoxyethyl-p-methoxy cinnamate, cyclohexyl-p-methoxy cinnamate, ethyl-$\alpha$-cyano-$\beta$-phenyl cinnamate, 2-ethylhexyl-a-cyano-β-phenyl cinnamate, glyceryl mono-2-ethylhexanoyl-diparamethoxy cinnamate; silicone-based cinnamic acid UV absorbants such as [3-bis(trimethylsiloxy)methylsilyl-1-methylpropyl]-3,4,5-trimethoxy cinnamate, [3-bis(trimethylsiloxy)methyl-silyl-3-methylpropyl]-3,4,5-trimethoxy cinnamate, [3-bis(trimethylsiloxy]methylsilylpropyl]-3,4,5-trimethoxy cinnamate, [3-bis(trimethylsiloxy)methyl-silylbutyl]-3,4,5-trimethoxy cinnamate, [3-tris(trimethylsiloxy)silylbutyl]-3,4,5-trimethoxy cinnamate, [3-tris(trimethylsiloxy)silylbutyl]-3,4,5-trimethoxy cinnamate, [3-tris(trimethylsiloxy)silyl-1-methylpropyl]-3,4-dimethoxy cinnamate; benzophenone-based UV absorbants such as 2,4-dihydroxybenzophenone, 2,2'-dihydroxy-4-methoxy-benzophenone, 2,2'-dihydroxy-4,4'-dimethoxybenzo-phenone, 2,2',4,4'-tetrahydroxybenzophenone, 2-hydroxyl-4-methoxybenzophenone, 2-hydroxy-4-methoxy-4'-methylbenzopheone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone; other UV absorbants such as 3-(4'methylbenzylidene)-d,1-camphor, 3-benzylidene-d,1-camphor, urocanic acid ethyl ester, 2-phenyl-5-methylbenzoxazole, 2,2'-hydroxy-5-methylphenylbenzo-triazole, 2-(2'-hydroxy-5'-t-octylphenyl)benzo-triazole, 2-(2'-hydroxy-5'-methylphpenyl)benzo-triazole, dibenzaladine, dianisoylmethane, 4-methoxy-4'-t-butyl dibenzoyl methane, 5-(3,3'-dimethyl-2-norbornylidene)-3-pentan-2-one; organic acids such as acylsarcosine acid (for example, sodium lauroylsarcosine), glutathione, citric acid, malic acid, tartaric acid, lactic acid; vitamin A and other derivatives, vitamin $B_6$ hydrochloride, vitamin $B_6$ tripalmitate, vitamin $B_6$ dioctanate, vitamin $B_2$ and its derivatives, vitamin $B_{12}$, vitamin $B_{15}$ and its derivatives, and other vitamin B's, α-tocopherol, β-tocopherol, γ-tocopherol, vitamin E acetate, and other vitamin E's, vitamin D's, vitamin H, pantotenic acid, pantethine, nicotic amide, benzyl nicotinate; and other vitamins; saponins such as γ-oryzanol, allantoin, glycyrrhizinic acid (salt), glycyrrhetinic acid and its derivatives, tranexamic acid and its derivatives [as a tranexamic acid derivative, a tranexamic acid dimer (for example, hydrochloride trans-4-(transaminomethylcyclohexanecarbonyl)aminomethylcyclohexa necarboxylic acid etc.), an ester of tranexamic acid and hydroquinone (for example, trans-4-aminomethylcyclohexane carboxylate-4'-hydroxyphenyl ester etc.), an ester of tranexamic acid and gentisic acid (for example, 2-(trans-4-aminomethylcyclohexyl-carbonyloxy)-5-hydroxybenzoic acid and its salts, etc.), an amide of tranexamic acid (for example, trans-4-aminomethylcyclohexanecarboxylic acid methylamide and its salts, trans-4-(P-methoxyvinzoyl)-aminomethylcyclohexanecarboxylic acid and its salts, trans-4-guanidinomethylcyclohexane carboxylic acid and its salts, etc.], hinokitiol, bisabolol, eucalypton, thymol, inositol, cyclosaponin, ginseng saponin, sponge gourd saponin, mukurossi peel saponin; various medicines such as panthenyl ethyl ether, ethynyl estradiol, tranexamic acid, albutin, cepharanthine, placenta extract; sorrel, sophora root, nuphar, orange, sage, yarrow, mallow, swertia herb, wild thyme, Japanese angelica root, bitter orange peel, birch, horsetail, sponge gourd, horse chestnut, saxifrage, arnica, lily, Japanese mugwort, peony root, aloe, gardenia, sawara cypress, and other plant extracts; colors; porous and/or hydroscopic powders (for example, powders of starches obtained from corn, potato, etc., silicic acid anhydride, talc, kaolin, aluminum magnesium silicate, and calcium alginate); and nonionic surfactants such as sorbitan monolaurate, sorbitan monopalmitate, sorbitan sesquioleate, sorbitan trioleate, polyoxyethylene sorbitan monolaurate, polyoxycetylene sorbitan monostearate, polyethyleneglycol monooleate, polyoxyethylene alkyl ether, polyglycol diether, lauroyl diethanol amide, fatty acid isopropanol amide, maltitol hydroxy fatty acid ether, alkylated polysaccharide, alkyl glucoside, sugar ester; cationic surfactants such as stearyl trimethyl ammonium chloride, chlorinated benzalkonium lauryl amino oxide; anionic surfactants such as sodium palmitate, sodium laurate, sodium laurate, potassium laurosulfate, alkyl sulfonate triethanol amine ether, Turkeyred oil, linear dodecyl benzene sulfuric acid, polyoxyethylene hydrogenated castor oil malic acid, acylmethyltaurin; bipolar surfactants; neutralizing agents; preservatives; fragrances; pigments, etc. may be mentioned.

The method of production of a W/O/W type emulsified cosmetic composition of the present invention is not particularly limited. The cosmetic composition may be produced by an ordinary method. For example, it may be obtained by preparing a W/O type emulsion produced by agitation and mixture using emulsification device such as a disperser and mixing and emulsifying it with an external aqueous phase including an alkyl-modified carboxyvinyl polymer.

EXAMPLES

The present invention will now be explained in further detail using Examples, but the present invention is not limited to these Examples in any way. Note that the weights of the formulations are "% by weight".

Before the Examples, the test methods and evaluation methods adopted in the Examples and Comparative Examples will be explained.

Organoleptic Test and Evaluation of Feeling in Use

A panel of 30 each men and women (total 60 persons) was asked to actually use samples obtained from the Examples and Comparative Examples. The feeling of phase inversion, refreshingness, smoothness, and moistness were evaluated based on the following criteria:

Criteria of Evaluation of Feeling of Phase Inversion

++: At least 30 of panel felt phase inversion
+: 10 to 29 of panel felt phase inversion
±: 5 to 9 of panel felt phase inversion
- : 4 or less of panel felt phase inversion

Criteria of Evaluation of Refreshingness

++: At least 30 of panel felt refreshingness of skin
+: 10 to 29 of panel felt refreshingness of skin
±: 5 to 9 of panel felt refreshingness of skin
-: 4 or less of panel felt refreshingness of skin

Criteria of Evaluation of Smoothness of Skin

++: At least 30 of panel felt smoothness of skin
+: 10 to 29 of panel felt smoothness of skin
±: 5 to 9 of panel felt smoothness of skin
-: 4 or less of panel felt smoothness of skin

Criteria of Evaluation of Moistness of Skin

++: At least 30 of panel felt moistness of skin
+: 10 to 29 of panel felt moistness of skin
±: 5 to 9 of panel felt moistness of skin
- : 4 or less of panel felt moistness of skin

Evaluation of Stability

The samples obtained in the Examples and Comparative Examples were filled in 50 ml sample tubes (glass bottles) and allowed to stand at room temperature and 50°C for 2 weeks, then were evaluated by the naked eye and microscope.

(Evaluation Criteria)

++: No separation at all. Emulsion particles stable.
+: No separation, but emulsion particles partially destroyed
±: No separation, but emulsion particles destroyed
- : Separation. Emulsion particles also destroyed.

Examples 1 to 7

Samples were prepared by the compositions shown in the following Tables I and II.

Process of Production

Ingredients (7) and (8) were dissolved in (12) (part), then (9) and (10) were added and completely dissolved to prepare an external aqueous phase part. Next, (1) was added to the oil phase (4) and (5) and the result homogeneously dissolved at 60°C. To this was added the aqueous solution obtained by adding (6) to (12) (part of remainder), then this was homogeneously dispersed to obtain a W/O type emulsion part. This W/O type emulsion part was added to the initially prepared external aqueous phase part, then the resultant product was homogeneously dispersed, then was neutralized with the solution of (11) in (12) (remainder) and the resultant product homogeneously dispersed.

Evaluation

These samples were used for evaluation of the feeling in use and stability according to the above-mentioned evaluation methods. Further, the types of emulsions were evaluated by the naked eye and by observation with a microscope. The results are shown in Table I and II.

Table 1

| Ingredients | Examples | | | |
|---|---|---|---|---|
| | 1 | 2 | 3 | 4 |
| **Formulation (wt%)** | | | | |
| (1) Polyethylene glycol dipolyhydroxystearate ("Arlacel P135", HLB about 5.5) | 2.0 | 0.1 | 5.0 | 1.0 |
| (2) POE (20) glyceryl monoisostearate (HLB=14) | - | - | - | - |
| (3) POE (6) glyceryl monoisostearate (HLB=8) | - | - | - | - |
| (4) Squalane | 10.0 | 10.0 | 10.0 | 10.0 |
| (5) Dimethyl polysiloxane | 10.0 | 10.0 | 10.0 | 10.0 |
| (6) Sodium L-glutamate | 0.5 | 0.05 | 1.0 | 0.5 |
| (7) Alkyl-modified carboxyvinyl polymer ("PEMULEN TR-1") | 0.1 | 0.1 | 1.0 | 0.01 |
| (8) Carboxyvinyl polymer | 0.4 | 0.4 | 0.4 | 0.4 |
| (9) Paraben | q.s. | q.s. | q.s. | q.s. |
| (10) 1,3-butylene glycol | 5.0 | 5.0 | 5.0 | 5.0 |
| (11) Potassium hydroxide | q.s. | q.s. | q.s. | q.s. |
| (12) Ion exchanged water | Bal. | Bal. | Bal. | Bal. |
| **Results of evaluation** | | | | |
| Feeling in use | | | | |
| Phase inversion | ++ | + | ++ | ++ |
| Refreshingness | ++ | ++ | + | ++ |
| Smoothness | ++ | ++ | ++ | ++ |
| Moistness | ++ | ++ | ++ | ++ |
| Emulsion type | W/O/W | W/O/W | W/O/W | W/O/W |
| Stability | | | | |
| Room temperature | + | + | + | + |
| 50°C | + | + | + | + |

Table II

| Ingredients | Examples | | |
|---|---|---|---|
| | 5 | 6 | 7 |
| Formulation (wt%) | | | |
| (1) Polyethylene glycol dipolyhydroxystearate ("Arlacel P135", HLB about 5.5) | 2.0 | 2.0 | 2.0 |
| (2) POE(20)glyceryl monoisostearate (HLB=14) | - | - | - |
| (3) POE(6)glyceryl monoisostearate (HLB=8) | - | - | - |
| (4) Squalane | 10.0 | 10.0 | 10.0 |
| (5) Dimethyl polysiloxane | 10.0 | 10.0 | 10.0 |
| (6) Sodium L-glutamate | 0.01 | 5.0 | 0.5 |
| (7) Alkyl-modified carboxyvinyl polymer ("PEMULEN TR-1") | 0.1 | 0.1 | 0.01 |
| (8) Carboxyvinyl polymer | 0.4 | 0.4 | 0.4 |
| (9) Paraben | q.s. | q.s. | q.s. |
| (10) 1,3-butylene glycol | 5.0 | 5.0 | 5.0 |
| (11) Potassium hydroxide | q.s. | q.s. | q.s. |
| (12) Ion exchanged water | Bal. | Bal. | Bal. |
| Results of evaluation | | | |
| Feeling in use | | | |
|   Phase inversion | ± | + | + |
|   Refreshingness | + | + | + |
|   Smoothness | + | + | + |
|   Moistness | + | + | + |
| Emulsion type | W/O/W | W/O/W | W/O/W |
| Stability | | | |
|   Room temperature | ± | ± | ± |
|   50°C | ± | ± | ± |

As shown in Tables I and II, Examples 1 to 7 all gave W/O/W type emulsions superior in feeling in use and superior in stability.

Comparative Examples 1 to 9

Samples were prepared by the compositions shown in the following Tables III and IV.

Process of Production

Comparative Example 1

Ingredients (7) and (8) were dissolved in (12) (part), then (9) and (10) were added and completely dissolved to prepare an aqueous phase part. Next, (12) (part of remainder) was added to the oil phase (4) and (5) and the resultant mixture homogeneously dispersed. This was added to the initial prepared external aqueous phase part, then the resultant product was homogeneously dispersed and neutralized by the solution of (11) in (12) (remainder) and the resultant product homogeneously dispersed.

Comparative Examples 2 to 8

Ingredients (7) and (8) (in Comparative Example 4, only (8)) were dissolved in (12) (part), then (9) and (10) were added and completely dissolved to prepare an aqueous phase part. Next, one of (1) to (3) was added to the oil phase (4) and (5) and the result homogeneously dispersed. To this was added the aqueous solution obtained by adding (6) (in Comparative Examples 2, 3, 5, and 7, (6) not added) to (12) (part of remainder) and the result was homogeneously dispersed. This was added to the initially prepared aqueous phase part and the resultant mixture homogeneously dispersed. This was further neutralized by the solution of (11) in (12) (remainder) and the resultant product homogeneously dispersed.

Comparative Example 9

Ingredient (8) were dissolved in (12) (part), then (2), (9), and (10) were added and completely dissolved to prepare an external aqueous phase part. Next, (1) was added to the oil phase (4) and (5) and the resultant mixture homogeneously dissolved at 60°C. To this was added the aqueous solution obtained by adding (6) to (12) (part of remainder), then the resultant mixture was homogeneously dispersed. This was added to the initial prepared external aqueous phase part, then the resultant mixture was homogeneously dispersed and neutralized by the solution of (11) in (12) (remainder) and the resultant product homogeneously dispersed.

Evaluation

These samples were used for evaluation of the feeling in use and stability according to the above-mentioned evaluation methods. Further, the types of emulsions were evaluated. The results are shown in Table III and IV.

Table III

| Ingredients | Comparative examples | | | |
|---|---|---|---|---|
| | 1 | 2 | 3 | 4 |
| Formulation (wt%) | | | | |
| (1) Polyethylene glycol dipolyhydroxystearate ("Arlacel P135", HLB about 5.5) | - | 0.01 | 2.0 | 2.0 |
| (2) POE (20) glyceryl monoisostearate (HLB=14) | - | - | - | - |
| (3) POE (6) glyceryl monoisostearate (HLB=8) | - | - | - | - |
| (4) Squalane | 10.0 | 10.0 | 10.0 | 10.0 |
| (5) Dimethyl polysiloxane | 10.0 | 10.0 | 10.0 | 10.0 |
| (6) Sodium L-glutamate | - | - | - | 0.5 |
| (7) Alkyl-modified carboxyvinyl polymer ("PEMULEN TR-1") | 0.1 | 0.1 | 0.1 | - |
| (8) Carboxyvinyl polymer | 0.4 | 0.4 | 0.4 | 0.4 |
| (9) Paraben | q.s. | q.s. | q.s. | q.s. |
| (10) 1,3-butylene glycol | 5.0 | 5.0 | 5.0 | 5.0 |
| (11) Potassium hydroxide | q.s. | q.s. | q.s. | q.s. |
| (12) Ion exchanged water | Bal. | Bal. | Bal. | Bal. |
| Results of evaluation | | | | |
| Feeling in use | | | | |
| Phase inversion | - | - | - | + |
| Refreshingness | + | ± | ± | ± |
| Smoothness | + | ± | ± | + |
| Moistness | ± | ± | ± | + |
| Emulsion type | O/W | O/W | O/W | W/O/W |
| Stability | | | | |
| Room temperature | + | + | + | - |
| 50°C | + | + | + | - |

Table IV

| Ingredients | Examples | | | | |
|---|---|---|---|---|---|
| | 5 | 6 | 7 | 8 | 9 |
| Formulation (wt%) | | | | | |
| (1) Polyethylene glycol dipolyhydroxystearate ("Arlacel P135", HLB about 5.5) | - | - | - | - | 2.0 |

Table IV   (continued)

| Ingredients | Examples | | | | |
|---|---|---|---|---|---|
| | 5 | 6 | 7 | 8 | 9 |
| (2) POE (20) glyceryl monoisostearate (HLB=14) | 2.0 | 2.0 | - | - | 2.0 |
| (3) POE (6) glyceryl monoisostearate (HLB=8) | - | - | 2.0 | 2.0 | - |
| (4) Squalane | 10.0 | 10.0 | 10.0 | 10.0 | 10.0 |
| (5) Dimethyl polysiloxane | 10.0 | 10.0 | 10.0 | 10.0 | 10.0 |
| (6) Sodium L-glutamate | - | 0.5 | - | 0.5 | 0.5 |
| (7) Alkyl-modified carboxyvinyl polymer ("PEMULEN TR-1") | 0.1 | 0.1 | 0.1 | 0.1 | - |
| (8) Carboxyvinyl polymer | 0.4 | 0.4 | 0.4 | 0.4 | 0.4 |
| (9) Paraben | q.s. | q.s. | q.s. | q.s. | q.s. |
| (10) 1,3-butylene glycol | 5.0 | 5.0 | 5.0 | 5.0 | 5.0 |
| (11) Potassium hydroxide | q.s. | q.s. | q.s. | q.s. | q.s. |
| (12) Ion exchanged water | Bal. | Bal. | Bal. | Bal. | Bal. |
| Results of evaluation | | | | | |
| Feeling in use | | | | | |
|   Phase inversion | - | - | - | - | + |
|   Refreshingness | ± | ± | ± | ± | ± |
|   Smoothness | ± | ± | ± | ± | ± |
|   Moistness | ± | ± | + | + | + |
| Emulsion type | O/W | O/W | O/W | O/W | W/O/W |
| Stability | | | | | |
|   Room temperature | + | + | + | + | - |
|   50°C | + | + | + | + | - |

As shown in Tables III and IV, Comparative Examples 1 to 3 and Comparative Examples 5 to 8 did not give W/O/W type emulsions. Further, Comparative Examples 4 and 9 gave W/O/W type emulsions, but these were very deficient in stability and the W/O/W type emulsion forms could not be sustained. Further, in all cases, the feeling in use could not be satisfied.

| Example 8: Moisturizing Cream | |
|---|---|
| Ingredient | wt% |
| (1) Glycerin | 5.0 |
| (2) Polyethylene glycol dipolyhydroxystearate (brandname "Arlacel P135", HLB about 5.5) | 3.0 |
| (3) Dimethyl polysiloxane | 5.0 |
| (4) Squalane | 10.0 |
| (5) Glyceryl tri (capryl/caprinate) | 5.0 |
| (6) Alkyl-modified carboxyvinyl polymer (brandname "PEMULEN TR-1") | 0.1 |
| (7) Carboxyvinyl polymer | 0.5 |
| (8) Paraben | q.s. |
| (9) Sodium L-glutamate | 1.0 |
| (10) Serine | 0.5 |
| (11) Arginine hydrochloride | 0.5 |
| (12) Potassium hydroxide | q.s. |
| (13) Ion exchanged water | Bal. |

Process of Production

Ingredients (6) and (7) were dissolved in (13), then (1) and (8) were added and completely dissolved to prepare an external aqueous phase part. Next, (2) was added to the oil phase (3) to (5) and the result homogeneously dissolved at 60°C. To this was added the aqueous solution obtained by adding (9) to (11) to (13) (part of remainder), then this was homogeneously dispersed to obtain a water-in-oil emulsion part. This water-in-oil emulsion part was added to the initially prepared external aqueous phase part, then the resultant mixture was homogeneously dispersed, then was neutralized by the solution of (12) in (13) (remainder) and the resultant product homogeneously dispersed.

| Example 9: Milky Lotion | |
|---|---|
| Ingredient | wt% |
| (1) Glycerin | 5.0 |
| (2) Polyethylene glycol dipolyhydroxystearate (brandname "Arlacel P135", HLB about 5.5) | 0.1 |
| (3) Cyclic silicone | 3.0 |
| (4) Octylmethoxy cinnamate | 2.0 |
| (5) Glyceryl tri(capryl/caprinate) | 1.0 |
| (6) Alkyl-modified carboxyvinyl polymer (brandname "PEMULEN TR-1") | 0.1 |
| (7) Keltrol | 0.1 |
| (8) Paraben | q.s. |
| (9) Sodium L-glutamate | 0.5 |
| (10) Sodium pyrocarboxylate | q.s. |
| (11) Albutin | q.s. |
| (12) Triethanol amine | q.s. |
| (13) EDTA | q.s. |
| (13) Ion exchanged water | Bal. |

Process of Production

Ingredients (6) and (7) were dissolved in (14) (part), then (1), (8), and (13) were added and completely dissolved to prepare an external aqueous phase part. Next, (2) was added to the oil phase (3) to (5) and the result homogeneously dissolved at 60°C. To this was added the aqueous solution obtained by adding (9) to (11) to (14) (part of remainder), then this was homogeneously dispersed to obtain a water-in-oil emulsion part. This water-in-oil emulsion part was added to the initially prepared external aqueous phase part, then the resultant mixture was homogeneously dispersed, then was neutralized by the solution of (12) in (14) (remainder) and the resultant product homogeneously dispersed.

| Example 10: Sun Screen | |
|---|---|
| Ingredient | wt% |
| (1) Glycerin | 5.0 |
| (2) Polyethylene glycol dipolyhydroxystearate (brandname "Arlacel P135", HLB about 5.5) | 1.0 |
| (3) Dimeticone copolyol | 1.0 |
| (4) Cyclic silicone | 10.0 |
| (5) Octylmethoxy cinnamate | 8.0 |
| (6) Glyceryl tri(capryl/caprinate) | 3.0 |
| (7) Alkyl-modified carboxyvinyl polymer (brandname "PEMULEN TR-1") | 0.2 |
| (8) Carboxyvinyl polymer | 0.3 |
| (9) Paraben | q.s. |
| (10) Sodium L-glutamate | 1.0 |
| (11) Trimethyl glycine | 0.5 |
| (12) Magnesium L-ascorbyl-2-phosphate | q.s. |
| (13) Potassium hydroxide | q.s. |
| (14) EDTA | q.s. |
| (15) Ion exchanged water | Bal. |

Process of Production

Ingredients (7) and (8) were dissolved in (15) (part), then (1), (9), and (14) were added and completely dissolved to prepare an external aqueous phase part. Next, (2) was added to the oil phase (4) to (6) and the result homogeneously dissolved at 60°C. To this was added the aqueous solution obtained by adding (10) to (12) to (15) (part of remainder), then this was homogeneously dispersed to obtain a water-in-oil emulsion part. This water-in-oil emulsion part was added to the initially prepared external aqueous phase part, then the resultant mixture was homogeneously dispersed, then was neutralized by the solution of (13) in (15) (remainder) and the resultant product homogeneously dispersed.

Examples 8 to 10 were all superior in feeling in use and stability.

INDUSTRIAL APPLICABILITY

As explained above, according to the present invention, a W/O/W type emulsified cosmetic composition having an excellent stability over time while having a refreshingness in use like a phase inversion, smoothness, and moistness when applied to the skin is provided.

**Claims**

1. A W/O/W type emulsified cosmetic composition comprising a W/O type emulsion dispersed in an external aqueous phase, wherein said W/O type emulsion contains an emulsifier having an HLB of not more than 7 and an electrolyte and wherein said external aqueous phase contains an alkyl-modified carboxyvinyl polymer.

2. A W/O/W type emulsified cosmetic composition as claimed in claim 1, wherein the emulsifier having an HLB of not more than 7 is at least one emulsifier selected from the group consisting of polyhydroxystearic acid esters of polyhydric alcohols and polyether-modified silicone-based surfactants.

3. A W/O/W type emulsified cosmetic composition as claimed in claim 1 or 2, wherein the electrolyte is at least one member selected from the group consisting of amino acids, L-ascorbic acids and derivatives thereof.

4. A W/O/W type emulsified cosmetic composition as claimed in any one of claims 1 to 3, wherein a content of the electrolyte is 0.01 to 5% by weight, based upon the total weight of the cosmetic composition.

5. A W/O/W type emulsified cosmetic composition as claimed in any one of claims 1 to 4, wherein a content of the emulsifier is 0.01 to 10% by weight, based upon the total weight of the cosmetic composition.

6. A W/O/W type emulsified cosmetic composition as claimed in any one of claims 1 to 5, wherein a content of the alkyl-modified carboxyvinyl polymer is 0.01 to 2% by weight, based upon the total weight of the cosmetic composition.

## INTERNATIONAL SEARCH REPORT

| International application No. |
| --- |
| PCT/JP02/02259 |

**A. CLASSIFICATION OF SUBJECT MATTER**
Int.Cl⁷ A61K7/00, 7/40, 7/42

According to International Patent Classification (IPC) or to both national classification and IPC

**B. FIELDS SEARCHED**

Minimum documentation searched (classification system followed by classification symbols)
Int.Cl⁷ A61K7/00, 7/40, 7/42

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 11-033391 A (Shiseido Co., Ltd.), 09 February, 1999 (09.02.99), Full text (Family: none) | 1-6 |
| Y | US 6149900 A (L'oreal S.A.), 21 November, 2000 (21.11.00), Claims; examples 1, 3, 5 & JP 11-180824 A & EP 908170 A1 & FR 2769224 A & DE 69800169 A | 1-6 |
| Y | JP 09-122476 A (NOF Corp.), 13 May, 1997 (13.05.97), Claims; examples (Family: none) | 1-6 |

☒ Further documents are listed in the continuation of Box C.   ☐ See patent family annex.

| | |
| --- | --- |
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br> 02 June, 2002 (02.06.02) | Date of mailing of the international search report<br> 18 June, 2002 (18.06.02) |
| Name and mailing address of the ISA/<br> Japanese Patent Office | Authorized officer |
| Facsimile No. | Telephone No. |

Form PCT/ISA/210 (second sheet) (July 1998)

13

**EP 1 369 101 A1**

<table>
<tr><td colspan="2">INTERNATIONAL SEARCH REPORT</td><td colspan="2">International application No.<br>PCT/JP02/02259</td></tr>
<tr><td colspan="4">C (Continuation).　DOCUMENTS CONSIDERED TO BE RELEVANT</td></tr>
<tr><td>Category*</td><td colspan="2">Citation of document, with indication, where appropriate, of the relevant passages</td><td>Relevant to claim No.</td></tr>
<tr><td>Y</td><td colspan="2">US 5840943 A  (Henkel Kommanditgesellschaft Auf Aktien),<br>24 November, 1998 (24.11.98),<br>Full text<br>& JP 10-501252 A        & WO 95/34528 A1<br>& EP 766661 A1          & DE 4420516 A</td><td>2</td></tr>
<tr><td>Y</td><td colspan="2">JP 2000-204276 A  (Shiseido Co., Ltd.),<br>25 July, 2000 (25.07.00),<br>Claims; examples<br>(Family: none)</td><td>2</td></tr>
<tr><td>Y</td><td colspan="2">JP 2000-154114 A  (Baiyarsdorf AG.),<br>06 June, 2000 (06.06.00),<br>Claims; examples<br>& EP 1002569 A1         & DE 19855153 A</td><td>2</td></tr>
</table>

Form PCT/ISA/210 (continuation of second sheet) (July 1998)